# EUROPÄISCHE PATENTSCHRIFT

(11) **EP 2 475 980 B1**
(45) Veröffentlichungstag und Bekanntmachung des Hinweises auf die Patenterteilung: **11.12.2013**
(21) Anmeldenummer: 10770704.4
(22) Anmeldetag: 09.09.2010
(51) Int. Cl.: G01N 29/036, G01N 33/86

(54) **VERFAHREN ZUR BESTIMMUNG DER GERINNUNGSZEIT**
METHOD FOR DETERMINING CLOTTING TIME
PROCÉDÉ DE DÉTERMINATION DU TEMPS DE COAGULATION

(30) Priorität: 09.09.2009 DE 102009040879
(43) Veröffentlichungstag der Anmeldung: 18.07.2012
(73) Patentinhaber: Andreas Hettich GmbH & Co. KG, 78532 Tuttlingen (DE)
(72) Erfinder: GEHRING, Frank, K., 72364 Obernheim (DE)
(74) Vertreter: Borchert, Uwe Rudolf
(86) Internationale Anmeldenummer: PCT/EP2010/005549
(87) Internationale Veröffentlichungsnummer: WO 2011/029601

(56) Entgegenhaltungen:
- WO-A2-2004/067130
- BERGLIN M ET AL: "The effect of substrate molecular mobility on surface induced immune complement activation and blood plasma coagulation", BIOMATERIALS, ELSEVIER SCIENCE PUBLISHERS BV., BARKING, GB, Bd. 25, Nr. 19, 1. August 2004 (2004-08-01), Seiten 4581-4590, XP004505468, ISSN: 0142-9612, DOI: DOI:10.1016/J.BIOMATERIALS.2003.11.050
- MUELLER L ET AL: "Quartz Crystal Microbalance with Sensitive Surface Coatings for Perioperative Bleeding Monitoring", TISSUE ENGINEERING PART A, Bd. 15, Nr. 3, März 2009 (2009-03), Seite 722, XP002612435, & 3RD CONGRESS ON REGENERATIVE BIOLOGY AND MEDICINE/3RD CONGRESS OF THE GERMAN-SOCIETY-FOR-STEM-CELL-R; STUTTGART, GERMANY; OCTOBER 09 -11, 2008 ISSN: 1937-3341
- NIMERI G ET AL: "Neutrophil interaction with protein-coated surfaces studied by an extended quartz crystal microbalance technique", COLLOIDS AND SURFACES B (BIOINTERFACES) ELSEVIER NETHERLANDS, Bd. 11, Nr. 5, 31. Juli 1998 (1998-07-31), Seiten 255-264, XP7916064, ISSN: 0927-7765
- WELLE ET AL: "Competitive protein adsorption on micro patterned polymeric biomaterials, and viscoelastic properties of tailor made extracellular matrices", BIOMOLECULAR ENGINEERING, ELSEVIER, NEW YORK, NY, US, Bd. 24, Nr. 1, 9. Februar 2007 (2007-02-09), Seiten 87-91, XP005881408, ISSN: 1389-0344, DOI: DOI:10.1016/J.BIOENG.2006.05.027

## Beschreibung

Die Erfindung betrifft ein Verfahren gemäß dem Oberbegriff des Anspruchs 1.

In der Notfallmedizin oder auch zur Überwachung von Patienten mit Gerinnungsstörungen ist der Gerinnungszustand bzw. die Gerinnungsfähigkeit des Blutes eines Patienten von großer Bedeutung. Gerinnungsstörungen haben meist gravierende Folgen für den Betroffenen. Die Folgen können sowohl bei zu hoher Gerinnungsneigung als auch bei zu geringer Gerinnungsneigung erheblich sein. Im Allgemeinen werden zur Beurteilung der Gerinnungsneigung Verfahren verwendet, die eine Bestimmung der sogenannten Quickzeit ermöglichen. Diese haben sich in der Historie unterschiedlich weiter entwickelt.

Eine besonders attraktive Methode bezüglich des technischen Potentials, ist die Gerinnungszeit mittels eines Schwingquarzes zu messen. Bei dieser Methode wird Blut oder Plasma auf die Schwingquarzoberfläche aufgebracht und das Frequenz- bzw. Dämpfungsverhalten über die Zeit gemessen. Sobald die Frequenz um einen gewissen Anteil abgefallen ist, schließt man auf die bis dahin gemessene Gerinnungszeit.

Ein Beispiel dafür ist die Schrift "Comparison of surface plasmon resonance and quartz crystal microbalance in the study of whole blood and plasma coagulation" (T.P. Viringe et al., Biosensors and Bioelectronics 15 (2000), S. 605-613). Diese Schrift zeigt grundsätzliche Möglichkeiten auf, Blutbestandteile mittels eines Schwingquarzes zu messen, hat allerdings den Nachteil, dass die Gerinnungszeit, also die Zeit von der Aktivierung des Blutes bzw. Plasmas bis zur Messung einer signifikanten Viskositätsänderung nicht genau bestimmt werden kann. Dadurch sind diese Verfahren relativ ungenau, da nie ein markanter Wert einer Kurve ermittelt wird, sondern lediglich eine Messung mit anschließender Datenverarbeitung die Bestimmung der Gerinnungszeit möglich ist.

Die Schrift "Acoustics of blood plasma on solid surface" offenbart eine Vorinkubation eines Schwingquarzes mit zitrathaltigen Plasma, um die unspezifischen Bindungsstellen abzusättigen. Dies hat zur Folge, dass die Anlagerung von Fibrin gemessen werden kann.

Die Schrift "The effect of substrate molecular mobility of surface induced immune complement activation and blood plasma coagulation" (M. Berglin et al., Biomaterials 25 (2004), S. 4581-4590) offenbart, das seine Gerinnungszeitmessung durch Messung der Frequenzänderung eines Schwingquarzes durchgeführt werden kann, indem zuerst Citratplasma zwei Minuten auf der Sensoroberfläche vorinkubiert wird und anschließend aktiviertes Plasma hinzgegeben wird.

Der Erfindung liegt die Aufgabe zugrunde, ein Verfahren anzugeben, mit dem es möglich ist den Zeitpunkt der Blutgerinnung schnell und exakt mittels eines Resonators in verbesserter Weise zu bestimmen.

In bekannter Weise wird der Schwingquarz mit einem Vorinkubationsfluid in Kontakt gebracht, bevor auf diesen ein Probenfluid appliziert wird. Dadurch bilden sich Ankerstellen aus, an welchen eine Gerinnung stattfinden soll. Durch die Vorinkubation lagern sich bereits Blutbestandteile an der adhäsiven Oberfläche des Resonators an. Auf diese Weise wird bereits eine Schicht aus Blutbestandteilen gebildet. Diese Schicht erleichtert im späteren Verfahren die Anlagerung weiterer Blutbestandteile, insbesondere die Ausbildung eines Fibrinnetzes, wodurch sich die Viskosität ändert. Somit wird gewährleistet, dass die Gerinnung gezielt in der Nähe der Schwingquarzoberfläche stattfindet und die von der Gerinnung verursachte Viskositätsänderung des Probenfluids zeitnah gemessen werden kann. Dieses Verfahren ermöglicht eine schnelle, genaue und vielseitige Bestimmung von plasmatischen Gerinnungsparametern mit nur einer Vorrichtung, die darüber hinaus ein hohes Potential zur Miniaturisierung und Automatisierung bietet.

Das Probenfluid kann vor der Applikation mit einem Aktivator versetzt werden, der die Gerinnung auslöst. Das aktivierte Probenfluid wird dann auf den vorinkubierten Schwingquarz aufgebracht, woraufhin die Schwingungsparameter des Resonators gemessen werden. Die Gerinnung findet dann nahe der Schwingquarzoberfläche statt, da sich die aktivierten Blutbestandteile an die durch die Vorinkubation entstandene Schicht anlagern.

Auf diese Weise ist eine Bestimmung der entsprechenden Gerinnungszeit binnen weniger Sekunden möglich. Ohne Vorinkubation des Resonators bestünde immer eine Unsicherheit in der Bestimmung der Gerinnungszeit, da der Ort der Gerinnung nicht beeinflusst werden kann. Die Unsicherheit rührt daher, dass sich die Viskosität zwar um den Gerinnungsstartpunkt verändert, aber aufgrund des Abstands zum Resonator von diesem noch nicht detektiert wird bzw. durch die möglichen Grenzschichten nicht detektierbar ist.

Insofern werden durch die Vorinkubation mit einem Blutbestandteile enthaltenden Fluid Eigenschaften der Adsorptionsmessung, die an sich parasitäre Effekte bei der Viskositätsmessung indizieren ausgenutzt, um eine Verbesserung der Genauigkeit bei der Gerinnungszeitbestimmung zu erreichen.

Erfindungsgemäß wird der Resonator nach der Vorinkubation gespült, insbesondere mit einer Pufferlösung. Durch Spülen des Resonators wird gewährleistet, dass überschüssige bzw. ungebundene Blutbestandteile, die sich an dem Resonator angelagert haben entfernt werden. Ferner kann die Oberfläche der Resonatoren nach diesem Schritt außerhalb der Messkammer trocken geblasen werden, was wiederum eine Lagerung der Resonatoren ermöglicht. Die Resonatoren können auf diese Weise zuerst vorinkubiert, und getrocknet werden und erst bei Bedarf in ein entsprechendes Messgerät eingesetzt werden.

Zudem stellen überschüssige, nicht gebundene Blutbestandteile in der Messkammer oder an der Resonatoroberfläche einen zusätzlichen Unsicherheitsfaktor dar. Indem überzählige Blutbestandteile abgewaschen werden, wird diese Störgröße eliminiert. indizieren ausgenutzt um eine Verbesserung der Genauigkeit bei der Gerinnungszeitbestimmung zu erreichen.

In einem weiteren Schritt kann der Resonator nach der Vorinkubation mit einer Pufferlösung gespült werden. Durch Spülen des Resonators wird gewährleistet, dass überschüssige bzw. ungebundene Blutbestandteile, die sich an dem Resonator angelagert haben entfernt werden. Ferner kann die Oberfläche der Resonatoren nach diesem Schritt außerhalb der Messkammer trocken geblasen werden, was wiederum eine Lagerung der Resonatoren ermöglicht. Die Resonatoren können auf diese Weise zuerst vorinkubiert und getrocknet werden und erst bei Bedarf in ein entsprechendes Messgerät eingesetzt werden.

Zudem stellen überschüssige, nicht gebundene Blutbestandteile in der Messkammer oder an der Resonatoroberfläche einen zusätzlichen Unsicherheitsfaktor dar. Indem überzählige Blutbestandteile abgewaschen werden, wird diese Störgröße eliminiert.

In besonders vorteilhafter Weise entspricht das Vorinkubationsfluid, mit dem der Resonator vorinkubiert wird, dem Probenfluid. Dies vermeidet eine Einschleusung von Gerinnungsfaktoren durch die Vorinkubation, die das Probenfluid möglicherweise nicht beinhaltet. Das Vonnkubationsftuid kann Vollblut, oder halbsynthetisch oder vollsynthetisch hergestelltes Blutbestandteile enthaltendes Fluid sein.

Blutbestandteile im Sinne der Erfindung sind insbesondere, gerinnungsrelevante Bestandteile, beispielsweie Thrombozyten und / oder Gerinnungsfaktoren.

Insbesondere wird als Schwingungsparameter die Resonanzfrequenz über die Zeit gemessen. Die Messung der Resonanzfrequenz eignet sich besonders gut zur Bestimmung einer Viskositätsänderung, da sich diese schon bei geringer Anlagerung merklich verändert Die Gerinnungszeit wird bestimmt, indem die Zeitspanne von der Aktivierung des Probenfluids bis zu einem lokalen Frequenzmaximum in der Resonanzfrequenzkurve gemessen wird. Alternativ dazu kann auch die Dämpfung als Schwingungsparameter gemessen werden. Die Charakteristik des Dämpfungsverlaufs ist dabei invers zu dem der Resonanzfrequenz.

Anstatt eines lokalen Maximums bei der Resonanzfrequenz, bzw. Minimum bei der Dämpfung, kann auch der Wendepunkt bestimmt werden. Ob der Wendepunkt oder ein Extrempunkt bestimmt wird ist abhängig von der Oberflächenbeschichtung des Resonators.

Aufgrund dieses Zeitwertes, also die Zeitspanne von der Aktivierung der Probe bis zur Gerinnung, kann abhängig vom zugegebenen Aktivator auf entsprechende Gerinnungsanomalien geschlossen werden.

Wird die Probe beispielsweise mittels Calciumthromboplastinlösung aktiviert entspricht die gemessene Zeitspanne bis zur Gerinnung der Thromboplastinzeit, die auch in der Literatur als Quickzeit bezeichnet wird. Die Ermittlung der Quickzeit ist beispielsweise für Marcumar-Patienten von großer Bedeutung. Ist die Quickzeit zu lange, besteht für diese die Gefahr innerer Blutungen.

Wird das Probenfluid anderseits mittels Kontaktphase und Phospholipiden aktiviert, entspricht die zugeordnete Zeitspanne der aPTT, welches eine weitere wichtige Größe in der heutzutage durchgeführten Gerinnungsdiagnostik darstellt

Ferner kann die Activated Clotting Time (ACT) gemessen werden. Die ACT wird bestimmt, indem das Probenfluid mit Kaolin oder Kieselerde aktiviert wird. Mittels der ACT in Kombination mit der Kenntnis der Thrombozytenzahl, kann eine Aussage über die Funktion des endogenen Gerinnungssystems und das Vorliegen einer disseminierten intravasalen Koagulopathie (DIC) getroffen werden.

Zusätzlich zur Bestimmung der Gerinnungszeit, kann die Kinetik der Frequenz und / oder der Dämpfungskurve vor allem aus der Funktion der Frequenz in Abhängigkeit der Dämpfung bestimmt werden. Dadurch kann zusätzlich eine Aussage über die Cloteigenschaften getroffen werden. Dadurch wird eine genauere Analyse der Gerinnung ermöglicht.

Als Probenfluid wird vorzugsweise Vollblut oder Plasma verwendet. Vollblut eignet sich vorrangig bei einer Analyse der Gerinnungszeit eines Notfallpatienten, Plasma für den Einsatz in der Forschung.

In besonders vorteilhafter Weise lässt sich eine Hyperfibrinolyse belegen. Dafür werden die Schwingungsparameter länger gemessen, als für die Gerinnungszeitbestimmung notwendig ist. Für die Messung der Gerinnungszeit ergibt sich ein Frequenzabfall respektive ein Dämpfungsanstieg. Bei einer Hyperfibrinolyse bzw. Fibrinolyse entwickelt sich dieser Wert wieder zurück.

Untersuchungen haben ergeben, dass es besonders vorteilhaft ist es den Resonator mindestens 15 Sekunden vorzuinkubieren.

Im Weiteren ist ein Verfahren zur Messung von Gerinnungsparameter nach dem Oberbegriff des Anspruchs 1 angegeben, durch welches die Hyperfibrinolyse bestimmbar ist, indem wenigstens ein Schwingungsparameter über den Zeitpunkt der Gerinnung hinaus gemessen wird, wobei auf eine Hyperfibrinolyse geschlossen wird, sofern nach dem Gerinnungszeitpunkt eine entgegengesetzte Charakteristik des Schwingungsparameterverlaufs zur Gerinnung detektiert wird.
Dies bedeutet, wenn als Schwingungsparameter die Frequenz beobachtet wurde, konnte ein Abfallen der Frequenz während der Gerinnung beobachtet werden. Steigt die Frequenz nach der Gerinnung wieder an, wird auf eine Hyperfibrinolyse geschlossen werden. Analog wird bei einem Abfallen der Dämpfungskurve nach der Gerinnung eine Hyperfibrinolyse diagnostiziert. Dieses Verfahren, kann im Anschluss an das Erfindungsgemäße Verfahren durchgeführt werden, wodurch sich enorme Kosten und Zeiteinsparungen ergeben.

Es ist, wie bereits ausgeführt, grundsätzlich bekannt, die Gerinnungszeit mittels eines Schwingquarzes der eine der Blutprobe zugewandten Oberfläche aufweist, zu bestimmen. Nach dem Stand der Technik besteht jedoch das Problem, dass bei einer völlig protein- beziehungsweise zellresistenten Oberfläche die Gerinnung nicht unmittelbar auf der Schwingquarzoberfläche stattfindet. In diesem Fall kann die durch die Gerinnung hervorgerufene Viskositätsänderung nicht oder zumindest nicht valide gemessen werden. Im Fall einer adhäsiven Oberfläche, ist die angelagerte Schicht so dick, dass der Resonator derart beeinflusst wird, dass keine Messung mehr möglich ist.

Besonders gut zur Durchführung des Verfahrens eignet sich ein Schwingquarz mit einer Oberfläche, die sowohl adhäsive als auch nicht-adhäsive Bereiche gegenüber den Blutbestandteilen aufweist.

Diese Kombination aus adhäsiver und nicht-adhäsiver Oberfläche hat den Vorteil, dass sich Blutbestandteile an den adhäsiven Bereichen anlagern und durch die Ausbildung von Aggregaten und Fibrinnetzen die nicht-adhäsiven Bereiche überspannen.

Dadurch erreicht man, entgegen dem Stand der Technik zuverlässig, dass die Gerinnung unmittelbar an der Schwingquarzoberfläche stattfindet und die durch die Gerinnung hervorgerufene Viskositätsänderung zu einem aussagekräftigen Sensorsignal führt. Dadurch ist eine zuverlässige und genaue Messung von Parametern der plasmatischen Blutgerinnung gewährleistet

In einer weiteren vorteilhaften Ausführungsform sind die nicht-adhäsiven Bereiche protein- und/oder zellresistent ausgebildet. Dies hat den Vorteil, dass sich Blutbestandteile nicht an dieser Oberfläche anlagern. Adhärieren zu viele Blutbestandteile, kann keine Viskositätsänderung mehr gemessen werden, da die Viskosität mit der Adhäsion überlagert ist.

In einer besonders vorteilhaften Ausführungsform sind die adhäsiven und nicht adhäsiven Bereiche mosaikförmig auf der Schwingquarzoberfläche angeordnet. Bei einer solchen Oberflächengestaltung können besonders genaue Messungen durchgeführt werden.

Insbesondere sind die adhäsiven Bereiche aus Gold und die nicht-adhäsiven Bereiche aus Polyethylen (PE) ausgebildet Eine Ausbildung der Oberfläche aus diesen Materialen, hat den Vorteil, dass sowohl Gold als auch Polyethylen (PE) in der Mikrosystemtechnik weitverbreitete Werkstoffe sind und daher gut bekannt und leicht zu verarbeiten sind. Ein weiterer Vorteil in der Verwendung einer Goldschicht besteht darin, dass diese gleichzeitig als Elektrode des Schwingquarzes dienen kann.

Insbesondere ist die Oberfläche des Schwingquarzes so aufgeteilt, dass die nicht adhäsiven Bereiche einen Anteil von mindestens 20 Prozent und maximal 90 Prozent der Gesamtfläche des Sensors einnehmen. In diesem Bereich werden die Besten Ergebnisse erzielt.

Einen weiter vorteilhaften Effekt kann man erzielen, indem ein Aktivator, wie beispielsweise Thrombin, bereits in die Oberfläche des Schwingquarzes integriert ist. Man erzielt dadurch den Vorteil, dass der Zeitpunkt von der Aktivierung der Probe bis zur Applikation auf den Schwingquarz besonders kurz, sogar zeitgleich erfolgt. Ferner erreicht man, dass die gesamte Vorrichtung einfacher gestaltet werden kann, da nicht versucht werden muss aktiviertes Blut möglichst schnell durch Kanalsysteme oder ähnlichem dem Resonator zuzuführen. Die Blutbestandteile adhärieren an der aktivierten Schicht und werden damit aktiviert, wodurch die plasmatische Gerinnung ausgelöst wird. Dadurch kann beispielsweise die Gerinnungszeit vom Zeitpunkt der Blutapplikation bis zur Gerinnung bestimmt werden.

Weitere Vorteile, Merkmale und Anwendungsmöglichkeiten der vorliegenden Erfindung ergeben sich aus der nachfolgenden Beschreibung in Verbindung mit dem in der Zeichnung dargestellten Ausführungsbeispiel. Die Erfindung wird im Folgenden anhand der Zeichnung näher beschrieben.

In der Beschreibung, in den Patentansprüchen und in der Zusammenfassung und den Zeichnungen werden die in der unten aufgeführten Liste der Bezugszeichen verwendeten Begriffe und zugeordnete Bezugszeichen verwendet. In der Zeichnung bedeuten:
- Fig. 1: eine Darstellung der Anlagerung der Blutbestandteile des Probenfluids an die des Vorinkubationsfluids;
- Fig. 2: eine Aufzeichnung einer Resonanzfrequenz zur Bestimmung der Quickzeit (PTT), und
- Fig. 3: eine Vorrichtung mit einer Schwingquarzoberfläche aus PE und Gold.

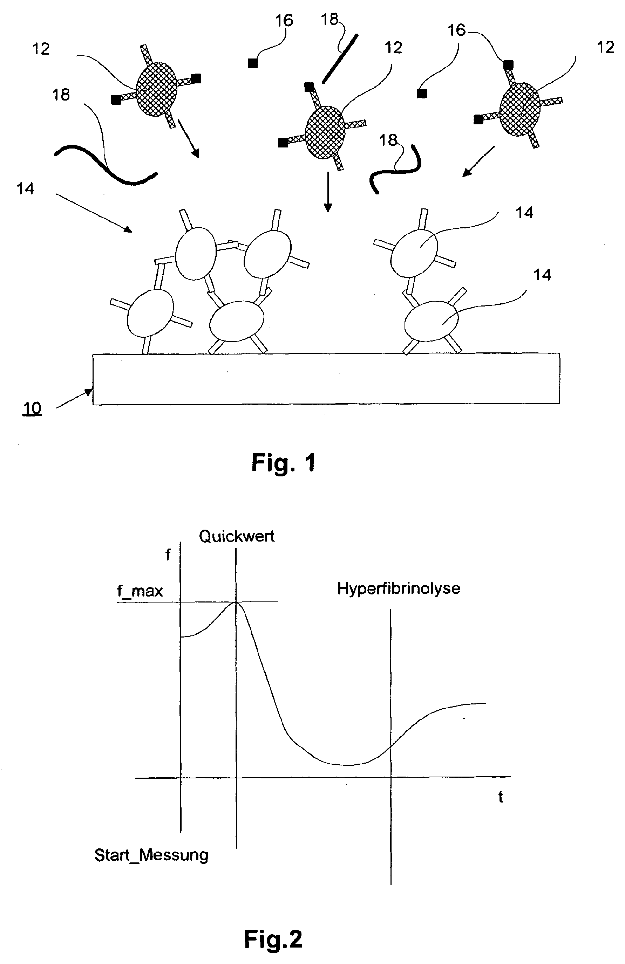

Fig. 1 zeigt einen vorinkubierte Oberfläche 10 eines Resonators, an dessen adhäsiver Oberfläche sich durch die Vorinkubation Blutbestandteile 14 des Inkubationsfluids angelagert haben. Ferner sind die Blutbestandteile 12 des aktivierten Probenfluids dargestellt. Der Aktivator 16 löst die Gerinnung aus.

Durch die bereits bestehenden Ankerstellen, die durch die Blutbestandteile 14 des Vorinkubafionsfluids zu Stande gekommen sind, ist das aktivierte Probenfluid bestrebt, an diesen Stellen, die sich unmittelbar an der Schwingquarzoberfläche befinden, den plasmatischen Gerinnungprozess z.B. durch Ankopplung von Fibrin auszulösen. Eine aktivierte plasmatische Gerinnung führt unter anderem durch die Ausbildung eines Fibrinnetzes zu einer Viskositätsänderung des Probenfluids direkt an der Schwingquarzoberfläche bzw. innerhalb der sensorisch wichtigen Eindringtiefe der akustischen Welle und kann somit sehr gut vom Resonator erfasst werden. Damit ist eine sehr schnelle und genaue Bestimmung des Gerinnungszeitpunktes möglich.

Fig. 2 zeigt die Frequenzkurve über die Zeit. Die Kurve startet zum Zeitpunkt der Applikation des Probenfluids. Die Gerinnung des Probenfluids hat stattgefunden, sobald ein lokales Maximum der Kurve erreicht ist Diese Zeitspanne entspricht abhängig vom Aktivator einer spezifischen Gerinnungszeit, in diesem Fall, bei einer Plasmathrombinlösung, der Quickzeit.

Im weiteren Verlauf der Kurve sieht man ein starkes Absinken der Frequenz. Diese gibt Rückschluss darauf, dass die Viskosität durch die Bildung von Fibrin weiter zunimmt Gegen Ende der Kurve nimmt die Frequenz wieder zu, was darauf hindeutet, dass eine Hyperfibrinolyse bzw. Fibrinolyse stattfindet, und somit das während der Gerinnung gebildete Fibrin wieder aufgelöst wird.

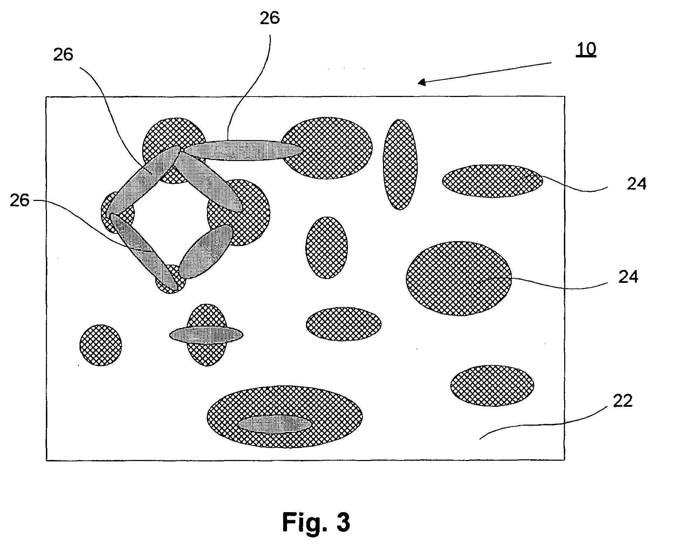

Fig. 3 zeigt die Oberfläche eines Schwingquarzes 20, die eine PE-Schicht 22 und eine Goldschicht 24 aufweist. Die PE-Schicht 22 ist in bekannter Weise zellresistent. Sie verhindert daher eine Anlagerung von Proteinen und anderen Zellbestandteilen und Blutbestandteile 26. Die Goldschicht hingegen ist adhäsiv und erlaubt daher eine Anlagerung von Blutbestandtteile 26 in diesem Bereich. Die Blutbestandteile 26 überspannen die nicht adhäsiven PE-Bereiche 22. Durch die geringen Anlagerungsmöglichkeiten wird eine Schichtdicke aus Blutbestandteilen gewährleistet, die eine Gerinnung direkt an der Oberfläche des Schwingquarzes erzielt Durch diese Anordnung, wird eine dünne Schicht aus Blutbestandteilen erreicht, die zum einen dafür verantwortlich ist, dass die Gerinnung nahe der Quarzoberfläche stattfindet, andererseits, die an der Oberfläche angelagerte Schicht nicht zu dick ist, um das Schwingungsverhalten des Sensors zu beeinflussen.

Mit Hilfe des vorgestellten Verfahrens können verschiedene Gerinnungszeiten gemessen werden. Durch die Verwendung eines Schwingquarzes, wird ein hohes Maß an Automationsfähigkeit und Miniaturisierungspotential.

### Bezugszeichenliste

- 10: Resonator
- 12: Blutbestandteile des Probenfluids
- 14: Blutbestandteile des Vorinkubationsfluids
- 16: Aktivator
- 18: Fibrin
- 20: Schwingquarz
- 22: PE-Schicht
- 24: Gold- Schicht
- 26: Blutbestandteile

## Patentansprüche

1. Verfahren zur Bestimmung der Gerinnungszeit eines, Blutbestandteile enthaltendem Probefluids mittels eines Resonators, dessen Schwingungsparameter gemessen werden, mithilfe derer die Viskositätsänderung des Probefluids bestimmt wird, wobei der Resonator eine wenigstens teilweise adhäsive Oberfläche (10) aufweist, die mit dem Probenfluid (12) in Kontakt steht, wobei eine Vorinkubation der Oberfläche (10) mit einem Blutbestandteile enthaltendem Vorinkubationsfluid (14) erfolgt, das eine bekannte Interaktionsweise mit dem Gerinnungssystem aufweist, wobei sich dadurch Ankerstellen an den adhäsiven Bereichen der Oberfläche ausbilden, **dadurch gekennzeichnet, dass** der Resonator nach der Vorinkubation gespült wird.

2. Verfahren nach Anspruch 1, **dadurch gekennzeichnet, dass** das Vorinkubationsfluid synthetisch oder halbsynthetisch hergestellt wird.

3. Verfahren nach Anspruch 1, **dadurch gekennzeichnet, dass** das Vorinkubationsfluid (14) dem Probenfluid (12) entspricht.

4. Verfahren nach einem der vorangegangenen Ansprüche, **dadurch gekennzeichnet, dass** dem Probefluid (12) ein Aktivator (16) zugegeben wird.

5. Verfahren nach einem der vorangehenden Ansprüche, **gekennzeichnet durch** die Applikation des aktivierten Probefluids auf den vorinkubierten Resonator.

6. Verfahren nach einem der vorangehenden Ansprüche, **dadurch gekennzeichnet, dass** der gemessene Schwingungsparameter der Resonanzfrequenz des Resonators entspricht.

7. Verfahren nach einem der vorangehenden Ansprüche, **dadurch gekennzeichnet, dass** die Gerinnungszeit einer Zeitspanne von der Aktivierung des Probenfluids bis zu einem lokalen Extremwert oder dem Wendepunkt des Verlaufs der Schwingungsparameter über die Zeit gemessen wird.

8. Verfahren nach Anspruch 5, **dadurch gekennzeichnet, dass** die Probe mittels Calcium-Thromboplastinlösung aktiviert wird und die Zeitspanne der Plasma-Thrombin-Zeit, Quickzeit entspricht.

9. Verfahren nach Anspruch 5, **dadurch gekennzeichnet, dass** die Probe mittels Kontaktphase und Phospholipiden aktiviert wird, und die Zeitspanne der aPTT entspricht.

10. Verfahren nach Anspruch 5, **dadurch gekennzeichnet, dass** die Probe mit Kaolin oder Kieselerde aktiviert wird, und die Zeitspanne der Activated Clotting Time (ACT) entspricht.

11. Verfahren nach einem der vorangehenden Ansprüche, **dadurch gekennzeichnet, dass** das Probenfluid (12) Vollblut, Plasma, oder Fibrinogen umfasst.

12. Verfahren nach einem der vorangehenden Ansprüche, **dadurch gekennzeichnet, dass** nach der Vorinkubation der Schwingquarz mit einer Pufferlösung gespült wird.

13. Verfahren nach einem der vorangehenden Ansprüche, **dadurch gekennzeichnet, dass** das Vorinkubationsfluid (14) synthetisch oder halbsynthetisch hergestellt wird und eine bekannte Interaktionsweise mit dem Gerinnungssystem aufweist.

14. Verfahren nach einem der vorangegangenen Ansprüche, **dadurch gekennzeichnet, dass** die Vorinkubationszeit mindestens 15 Sekunden beträgt.

15. Verfahren zur Messung von Gerinnungsparametem nach dem Oberbegriff des Anspruchs 1, **dadurch gekennzeichnet, dass** die Hyperfibrinolyse bestimmbar ist, indem wenigstens ein Schwingungsparameter über den Zeitpunkt der Gerinnung hinaus gemessen wird, wobei, sofern nach dem Gerinnungszeitpunkt ein invertierter Verlauf des Schwingungsparameters detektiert wird.

16. Verfahren nach einem der Ansprüche 1 bis 15, wobei ein Resonator verwendet wird, dessen Oberfläche (10) adhäsive Bereiche (24) und nicht-adhäsive Bereiche (22) gegenüber Blutbestandteilen aufweist.

17. Verfahren nach Anspruch 16, wobei ein Resonator verwendet wird, bei welchem die nicht adhäsiven Bereiche (22) möglichst protein- bzw. zellresistent ausgebildet sind.

18. Verfahren nach Anspruch 16 oder 17, wobei ein Resonator verwendet wird, dessen nicht-adhäsiven Bereiche (22) und die adhäsiven Bereiche (24) mosaikförmig auf der Schwingquarzoberfläche angeordnet sind.

19. Verfahren nach einem der Ansprüche 16 bis 18, wobei ein Resonator verwendet wird, bei dem die adhäsiven Bereiche aus Gold (24), die nicht-adhäsiven Bereiche aus Polyethylen (PE) (22) ausgebildet sind.

20. Verfahren nach einem der Ansprüche 16 bis 19, wobei ein Resonator verwendet wird, bei dem die nicht-adhäsiven Bereiche (22) einen Anteil von mindestens 20% und maximal 90% an der Gesamtfläche einnehmen.

21. Verfahren nach einem der Ansprüche 16 bis 20, wobei ein Resonator verwendet wird, bei dem ein Aktivator in die Oberfläche integriert ist.

22. Verfahren nach Anspruch 21, wobei ein Resonator verwendet wird, bei welchem die Oberfläche Fibrinogen aufweist.

## Claims

1. A method for determining the coagulation time of a sample fluid containing blood components by means of a resonator whose vibration parameters are measured and then used as a basis for determining the sample fluid viscosity change, which resonator has an at least partially adhesive surface (10) contacting said sample fluid (12), which surface (10) is preincubated with a preincubation fluid (14) containing blood components, which preincubation fluid (14) has a known way of interacting with the coagulation system, thus resulting in anchoring sites to be formed on the adhesive areas of said surface **characterized in that** said resonator is rinsed after preincubation.

2. The method of claim 1 **characterized in that** said preincubation fluid is produced synthetically or semi-synthetically.

3. The method of claim 1 **characterized in that** said preincubation fluid (14) corresponds to said sample fluid (12).

4. The method of one of the preceding claims **characterized in that** an activator (16) is added to said sample fluid (12).

5. The method of one of the preceding claims **characterized by** the application of said activated sample fluid to said preincubated resonator.

6. The method of one of the preceding claims **characterized in that** the measured vibration parameter corresponds to the resonance frequency of the resonator.

7. The method of one of the preceding claims **characterized in that** the coagulation time corresponds to and is measured as a time period from the activation of the sample fluid to a local extreme value or the inflection point of the curve of the vibration parameters over time.

8. The method of claim 5 **characterized in that** said sample is activated using a calcium thromboplastin solution and that said time period corresponds to the plasma thrombin time, i.e. Quick's time.

9. The method of claim 5 **characterized in that** said sample is activated by means of a contact phase and phospholipids, and that said time period corresponds to the aPTT.

10. The method of claim 5 **characterized in that** said sample is activated using kaolin or silica, and that said time period corresponds to the activated clotting time (ACT).

11. The method of one of the preceding claims **characterized in that** said sample fluid (12) comprises whole blood, plasma, or fibrinogen.

12. The method of one of the preceding claims **characterized in that** following preincubation, the vibrating quartz crystal is rinsed with a buffer solution.

13. The method of one of the preceding claims **characterized in that** said preincubation fluid (14) is produced synthetically or semi-synthetically and has a known way of interacting with the coagulation system.

14. The method of one of the preceding claims **characterized in that** said preincubation time is at least 15 seconds.

15. A method for measuring coagulation parameters according to the preamble of claim 1 **characterized in that** hyperfibrinolysis can be determined by measuring at least one vibration parameter beyond the coagulation time, provided that an inverted curve of the vibration parameter is detected after the coagulation time.

16. The method of one of claims 1 to 15 in which a resonator is used whose surface (10) has adhesive areas (24) and non-adhesive areas (22) with respect to blood components.

17. The method of claims 16 or 17 in which a resonator is used whose non-adhesive areas (22) have been formed to be as protein- and/or cell-resistant as possible.

18. The method of claims 16 or 17 in which a resonator is used whose non-adhesive areas (22) and adhesive areas (24) are arranged in the form of a mosaic on the vibrating quartz crystal surface.

19. The method of one of claims 16 to 18 in which a resonator is used whose adhesive areas are made of gold (24) whereas its non-adhesive areas are made of polyethylene (PE) (22).

20. The method of one of claims 16 to 19 in which a resonator is used whose non-adhesive areas (22) occupy between a minimum of 20% and a maximum of 90% of the total surface.

21. The method of one of claims 16 to 20 in which a resonator is used which has an activator incorporated in its surface.

22. The method of claim 21 in which a resonator is used whose surface includes fibrinogen.

## Revendications

1. Procédé de détermination du temps de coagulation d'un échantillon de fluide contenant des composants sanguins au moyen d'un résonateur dont le paramètre d'oscillation est mesuré et à l'aide duquel le changement de viscosité de l'échantillon de fluide est déterminée, le résonateur présentant une surface (10) au moins partiellement adhésive avec laquelle l'échantillon de fluide entre en contact (12), où la pré-incubation de la surface (10) est effectuée au moyen d'un fluide de pré-incubation (14) contenant des composants sanguins et présentant une interaction connue avec le système de coagulation, ce qui provoque la formation de points d'ancrage à la partie adhésive de la surface, **caractérisé en ce que** le résonateur est nettoyé à la fin de la pré-incubation.

2. Procédé selon la revendication 1, **caractérisé en ce que** le fluide de pré-incubation est produit de manière synthétique ou semi-synthétique.

3. Procédé selon la revendication 1, **caractérisé en ce que** le fluide de pré-incubation (14) correspond à l'échantillon de fluide (12).

4. Procédé selon l'une des revendications précédentes, **caractérisé en ce qu'**un activateur (16) est ajouté à l'échantillon de fluide (12).

5. Procédé selon l'une des revendications précédentes, **caractérisé en ce que** le fluide activé est appliqué sur le résonateur pré-incubé.

6. Procédé selon l'une des revendications précédentes, **caractérisé en ce que** le paramètre d'oscillation mesuré correspond à la fréquence de résonance du résonateur.

7. Procédé selon l'une des revendications précédentes, **caractérisé en ce que** le temps de coagulation est mesuré sur une période allant de l'activation de l'échantillon de fluide à une valeur extrême ou au point d'inflexion du cours du paramètre d'oscillation.

8. Procédé selon la revendication 5, **caractérisé en ce que** l'échantillon est activé au moyen d'une solution de thromboplastine calciqué et correspond à la période du temps de thrombine plasmatique, le taux de prothrombine.

9. Procédé selon la revendication 5, **caractérisé en ce que** l'échantillon est activé avec une phase de contact et des phospholipides et correspond à la période de l'APTT.

10. Procédé selon la revendication 5, **caractérisé en ce que** l'échantillon est activé avec du kaolin ou de la silice et que la période correspond au Activated Clotting Time (ACT).

11. Procédé selon l'une des revendications précédentes, **caractérisé en ce que** l'échantillon de fluide (12) comprend du sang entier, du plasma ou des fibrinogènes.

12. Procédé selon l'une des revendications précédentes, **caractérisé en ce que** le quartz est rincé à l'aide d'une solution tampon après la pré-incubation.

13. Procédé selon l'une des revendications précédentes, **caractérisé en ce que** le fluide de pré-incubation (14) est produit de façon synthétique ou semi-synthétique et interagit avec le système de coagulation d'une façon connue.

14. Procédé selon l'une des revendications précédentes, **caractérisé en ce que** le temps de pré-incubation est d'au moins 15 secondes.

15. Procédé de mesure du temps de coagulation d'après le terme générique de la revendication 1, **caractérisé en ce que** l'hyperfibrinolyse peut être déterminée en mesurant au moins un paramètre d'oscillation au-delà du moment de coagulation, dans la mesure où le paramètre d'oscillation suit un cours inverse après le moment de coagulation.

16. Procédé selon l'une des revendications 1 à 15, **caractérisé en ce qu'**un résonateur, dont la surface (10) présente des parties adhésives (24) et non adhésives (22) vis-à-vis des composants sanguins, est utilisé.

17. Procédé selon la revendication 16, **caractérisé en ce qu'**un résonateur, dont les surfaces non adhésives (22) sont formées de façon à résister le plus possible aux protéines ou cellules, est utilisé.

18. Procédé selon les revendications 16 à 17, **caractérisé en ce qu'**un résonateur, dont les surfaces non adhésives (22) et adhésives (24) sont disposées sur la surface du quartz en forme de mosaïque, est utilisé.

19. Procédé selon les revendications 16 à 18, **caractérisé en ce qu'**un résonateur, dont les surfaces adhésives sont faites en or (24) alors que les surfaces non adhésives sont faites en polyéthylène (PE) (22), est utilisé.

20. Procédé selon les revendications 16 à 19, **caractérisé en ce qu'**un résonateur, dont la surface non adhésive (22) forme de 20 à 90 % de la surface totale, est utilisé.

21. Procédé selon les revendications 16 à 20, **caractérisé en ce qu'**un résonateur, dans la surface duquel est intégré un activateur, est utilisé.

22. Procédé selon la revendication 21, au cours duquel est utilisé un résonateur dont la surface présente de la fibrinogène.
